# EUROPÄISCHE PATENTANMELDUNG

(11) **EP 2 354 017 A1**
(43) Veröffentlichungstag der Anmeldung: **10.08.2011**
(21) Anmeldenummer: 11153231.3
(22) Anmeldetag: 03.02.2011
(51) Int. Cl.: B65B 55/10, A61L 2/28, A61L 2/20

(54) **Packmittelbehandlungsanlage mit Messung des Sterilisationsmediums**

(30) Priorität: 04.02.2010 DE 102010006919
(71) Anmelder: Krones AG, 93073 Neutraubling (DE)
(72) Erfinder: Söllner, Jürgen, 93176 Beratzhausen (DE); Dahmen, Michael, 93055 Regensburg (DE)
(74) Vertreter: Bittner, Bernhard

(57) **Zusammenfassung**

Die Erfindung betrifft eine Vorrichtung (1) zur Behandlung von Packmitteln (20) mit einem Behandlungsraum (2), der gegenüber seiner Umgebung wenigstens teilweise abgetrennt ist, mit einer Zuführeinrichtung, welche dem Behandlungsraum Wasserstoffperoxid (H₂O₂) zuführt und mit einer Sensoreinrichtung (10), welche wenigstens einen für das Wasserstoffperoxid (H₂O₂) relevanten Parameter erfasst, wodurch die Sensoreinrichtung (10) derart mit dem Behandlungsraum (2) in Strömungsverbindung bringbar ist, dass der Parameter für das in dem Behandlungsraum (2) befindliche Wasserstoffperoxid erfassbar ist.

## Beschreibung

Die vorliegende Erfindung bezieht sich auf eine Vorrichtung zum Behandeln von Packmitteln, insbesondere von Behältnissen und/oder Behältnisverschlüssen. Aus dem Stand der Technik sind die unterschiedlichsten Vorrichtungen zum Behandeln von Packmitteln bekannt wie beispielsweise Sterilisationsanlagen zum Sterilisieren von Behältnissen, Füllanlagen zum Befüllen von Behältnissen, Reinigungsanlagen zum Reinigen von Behältnissen und dergleichen. Teilweise ist es dabei erforderlich, dass die Behältnisse unter sterilen Bedingungen abgefüllt werden wie beispielsweise im Falle von Fruchtsäften. Dabei wird üblicherweise eine gegenüber der Umgebung abgetrennte Kammer, die im Folgenden auch als Isolator bezeichnet wird, verwendet, innerhalb derer die Behältnisse entsprechend behandelt werden. Dabei ist es auch bekannt, dass bei derartigen Maschinen zur Sterilisation Wasserstoffperoxid (H₂O₂) eingesetzt wird. Bei derartigen Maschinen, welche H₂O₂ einsetzen wird in diesem Isolator zur Sterilisation des Isolators selbst oder auch zur Sterilisation von Behältnissen, wie beispielsweise Flaschen, Bechern, Kartonverpackungen oder Dosen oder deren Verschlüssen oder von Packstoffbahnen für Kartonverpackungen, Pouches, Standbeuteln oder Verschlusssiegelfolien eine H₂O₂ - Gasatmosphäre erzeugt.

Von besonderer Bedeutung für die Qualität der Sterilisation ist hierbei auch die Konzentration des Wasserstoffperoxids in der Gasatmosphäre in dem Isolator. Bei derzeitigen im Stand der Technik bekannten Anlagen wird eine Überwachung dieser Konzentration oder anderer wesentlicher Parameter für das Wasserstoffperoxid nicht vorgesehen.

Grundsätzlich sind zwar derartige Messgeräte zur Messung der H₂O₂ - Konzentration verfügbar, diese sind jedoch üblicherweise nicht flüssigkeitsbeständig und können insbesondere durch aggressive Chemikalien bei der Messung beeinträchtigt oder sogar zerstört werden. Hierbei ist zu berücksichtigen, dass der Isolator teilweise auch mit Flüssigkeiten wie heißer Lauge oder warmer Säure gereinigt wird.

Aus der EP 1 572 540 B1 ist eine Steuerung für eine Sterilisationsvorrichtung bekannt. Dabei wird ein Sterilisationsmittel während des gesamten Sterilisationsverfahrens in der gasförmigen Phase vorgehalten und es ist ein Konzentrationsmesser zum Messen der Konzentration des Sterilisationsmittels in dem Sterilisationsbereich vorgesehen, wobei eine Steuereinheit zum Steuern der Menge des in dem Sterilisationsbereich eingeleiteten Sterilisationsmittels auf der Grundlage der von dem Konzentrationsmesser gemessenen Konzentration vorgesehen ist. Ein besonderer Schutz dieser Konzentrationsmesseinrichtung wird jedoch in dieser Druckschrift nicht dargestellt.

Die US 5,167 927 beschreibt eine Messeinrichtung zum Durchführen von Messungen an Wasserstoffperoxid, wobei diese Messeinrichtung für Sterilisationseinrichtungen zum Sterilisieren von Packungen verwendbar ist.

Der vorliegenden Erfindung liegt daher die Aufgabe zugrunde, eine Überwachung von Sterilisationsmitteln in Isolatoren zu ermöglichen. Dabei soll diese Überwachung auch möglichst benutzerfreundlich ermöglicht sein.

Dies wird erfindungsgemäß durch die Gegenstände der unabhängigen Ansprüche erreicht. Vorteilhafte Ausführungsformen und Weiterbildungen sind Gegenstand der Unteransprüche.

Eine erfindungsgemäße Vorrichtung zur Behandlung von Packmitteln weist einen Behandlungsraum auf, der gegenüber seiner Umgebung wenigstens teilweise abgetrennt ist. Weiterhin weist die Vorrichtung eine Zuführeinrichtung auf, welche dem Behandlungsraum ein Sterilisationsmittel und insbesondere Wasserstoffperoxid zuführt. Daneben ist eine Sensoreinrichtung vorgesehen, welche wenigstens einen für das in den Behandlungsraum vorgesehene Wasserstoffperoxid relevanten Parameter erfasst.

Erfindungsgemäß ist die Sensoreinrichtung derart mit dem Behandlungsraum in Strömungsverbindung bringbar, dass der Parameter für das in dem Behandlungsraum befindliche Wasserstoffperoxid erfassbar ist. Unter einer Strömungsverbindung wird dabei insbesondere eine Verbindung bzw. Leitung verstanden, entlang derer ein gasförmiges Medium strömen kann.

Es wird daher vorgeschlagen, eine Sensoreinrichtung in der Art vorzusehen, dass diese einerseits zwar vor Reinigungsmitteln geschützt ist, andererseits jedoch in der Lage ist, den besagten Parameter für das Wasserstoffperoxid in dem Behandlungsraum zu bestimmen. Vorteilhaft ist die Sensoreinrichtung von dem Behandlungsraum außerhalb des Behandlungsraums angeordnet. Die Erfindung wird hier unter Bezugnahme auf Wasserstoffperoxid als Sterilisationsmittel beschrieben, die Erfindung kann jedoch auch für andere Sterilisationsmittel und insbesondere solche Sterilisationsmittel, welche als Gas oder Dampf vorliegen, Anwendung finden.

Vorzugsweise steht die Sensoreinrichtung über eine Verbindungsleitung mit dem Behandlungsraum in Verbindung. So ist es beispielsweise möglich, dass die Sensoreinrichtung selbst außerhalb des Behandlungsraums angeordnet ist, jedoch über eine Leitungsverbindung mit dem Behandlungsraum in Verbindung steht, so dass über diese Leitungsverbindung auch der relevante Parameter erfasst werden kann. Besonders bevorzugt ist die Verbindungsleitungslänge so kurz wie durch die mechanische Einbausituation bedingt möglich.

Es wäre jedoch auch möglich, die Sensoreinrichtung innerhalb eines Teilgehäuses in dem Behandlungsraum anzuordnen und dieses Gehäuse wiederum über die besagte Verbindungsleitung mit dem Behandlungsraum zu verbinden.

Bei einer weiteren vorteilhaften Ausführungsform ist - insbesondere zwischen der Sensoreinrichtung und dem Behandlungsraum - eine Absperreinrichtung zum wenigstens teilweise Absperren der Strömungsverbindung zwischen dem Behandlungsraum und der Sensoreinrichtung vorgesehen. Es wäre jedoch auch möglich, dass die Absperreinrichtung am Ende der Verbindungsleitung zum Trennen der Verbindung zwischen dem Behandlungsraum und der Sensoreinrichtung vorgesehen ist.

Vorteilhaft handelt es sich bei dieser Absperreinrichtung um ein Ventil, welches insbesondere steuerbar ist. Die Steuerung kann auf Maschinenebene oder auch auf Anlagenebene geschehen und an Ereignisse wie Reinigung, Sterilisation, Produktion oder auch Anlagenstillstand gekoppelt sein. Alternativ kann der Absperr-/Öffnungsvorgang durch einen Bediener ausgelöst werden. Es wäre jedoch auch möglich, dass diese Absperreinrichtung auf bestimmte physikalische Parameter beispielsweise das Auftreten von Flüssigkeit in dem Behandlungsraum reagiert. Anstelle eines Ventils wäre es jedoch auch möglich, eine Absperrklappe zu verwenden, welche beispielsweise in Form eines Deckels oder einer Klappe ausgestaltet ist, der/die die besagte Verbindungsleitung verschließt. Vorteilhaft ist die Auflagefläche der Absperreinrichtung mit einer geeigneten Dichtung versehen. Dabei können zur Betätigung jegliche geeigneten Antriebe zur Anwendung kommen, die eine ausreichende Anpressung der Absperreinrichtung an die Öffnung der Verbindungsleitung gewährleisten, wie beispielsweise pneumatische Antriebe, Elektromotoren und dergleichen. Daneben wäre es auch möglich, dass in der Verbindungsleitung zwischen der Sensoreinrichtung beispielsweise ein Siphon angeordnet ist, der zwar eine Strömungsverbindung zwischen dem Behandlungsraum und der Sensoreinrichtung hinsichtlich des gasförmigen H₂O₂ erlaubt, jedoch das Durchdringen von Flüssigkeiten aus dem Behandlungsraum zu der Sensoreinrichtung unterbindet.

Es wird daher vorgeschlagen, dass die Sensoreinrichtung beispielsweise vor Lauge, Säure und Wasser geschützt wird, indem sie beispielsweise hinter ein Sitzventil eingebaut wird. Dieses Ventil kann geschlossen sein, wenn der Isolator gereinigt wird und wird erst dann geöffnet, wenn eine H₂O₂ Messung erforderlich ist, so zum Beispiel in den Betriebszuständen Anlagensterilisation oder Produktion. Auf diese Weise kann eine kostengünstige und praktikable Messung im Isolator realisiert werden.

Daneben ist es jedoch auch denkbar, dass über eine Verrohrung verschiedene Maschinen beispielsweise Verschlussdesinfektion und Sterilisator mittels einer Sensoreinrichtung überwacht werden. Auch hier können mehrere Ventileinrichtungen vorgesehen sein, welche entsprechend die Verbindungen zwischen der Sensoreinrichtung und den einzelnen Maschinen öffnen bzw. schließen.

Vorteilhaft sind insbesondere bei einer Verwendung einer Sensoreinrichtung für verschiedene Maschinen die Verbindungsleitungen mit einem oder mehreren Spülmedienanschlüssen zu versehen, so dass die Verbindungsleitungen zwischen einzelnen Messvorgängen mit einem Gas, insbesondere einem sterilen Gas, bevorzugt mit Sterilluft gespült werden können. Es kann auch ein Inertgas wie beispielsweise Stickstoff verwendet werden. Dadurch ist das Risiko einer Verfälschung des Messergebnisses durch H2O2-Reste aus der vorherigen Messung minimiert.

Vorteilhaft handelt es sich bei dem Parameter für das Wasserstoffperoxid um eine Konzentration des Wasserstoffperoxids. Dies bedeutet, dass die Sensoreinrichtung insbesondere dazu dient, um eine Konzentration des Wasserstoffperoxids in dem Behandlungsraum zu messen.

Es könnten jedoch alternativ oder zusätzlich auch andere Parameter gemessen werden wie beispielsweise ein Druck oder eine Temperatur oder ein Volumenstrom oder eine Strömungsgeschwindigkeit des Sterilisationsmittels und dergleichen.

Vorteilhaft ist dabei die Sensoreinrichtung an einer Außenwandung des Behandlungsraums angeordnet. Weiterhin kann die Vorrichtung auch in einem Leitungssystem, beispielsweise einer Lüftungsanlage angeordnet sein. Es wird darauf hingewiesen, dass im erfindungsgemäßen Verständnis der Behandlungsraum das Volumen einer Lüftungsanlage und deren Leitungen mit einschließt.

Bei einer weiteren vorteilhaften Ausführungsform dient der Behandlungsraum zur Aufnahme von Behältnissen oder Behältnisverschlüssen. Dies bedeutet, dass in dem Behandlungsraum Behältnisse oder Behältnisverschlüsse behandelt werden, beispielsweise befüllt und/oder verschlossen werden oder gereinigt, gespült oder auch desinfiziert werden.

Vorteilhaft weist die Vorrichtung eine Transporteinrichtung auf, welche die Behältnisse innerhalb des Behandlungsraums transportiert. Während dieses Transports können die Behältnisse beispielsweise mit einer Flüssigkeit wie einem Getränk befüllt werden.

Bei einer weiteren vorteilhaften Ausführungsform weist die Vorrichtung eine Zuführeinrichtung zum Zuführen eines Reinigungsmediums auf, wobei sich dieses Reinigungsmedium von dem Wasserstoffperoxid unterscheidet. Dieses Reinigungsmedium dient dabei insbesondere der Innenraumreinigung des Behandlungsraums. Bei dieser Ausführungsform ist daher vorzugsweise eine so genannte CIP (Cleaning in place) Reinigung vorgesehen, um den Behandlungsraum zu reinigen. Daneben oder anstelle dessen kann jedoch auch eine Reinigung der Transporteinrichtung und sonstiger Behandlungseinheiten für die Behältnisse vorgesehen sein.

Die vorliegende Erfindung bezieht sich weiterhin auf eine Anlage zur Behandlung von Behältnissen mit einer Vorrichtung der oben beschriebenen Art sowie einer zweiten Behandlungsvorrichtung, wobei die Sensoreinrichtung auch zur Bestimmung eines Parameters für das der zweiten Behandlungsvorrichtung befindliche Wasserstoffperoxid dient. Bei dieser Ausführungsform dient wie oben erwähnt die Sensoreinrichtung zur Überprüfung zweier oder mehrerer Behandlungsanlagen. Dabei kann die Sensoreinrichtung über je eine Verbindungsleitung mit den beiden Behandlungseinrichtungen in (Strömungs)-verbindung stehen

Die vorliegende Erfindung ist weiterhin auf ein Verfahren zur Wasserstoffperoxidbestimmung gerichtet, wobei mittels einer Sensoreinrichtung wenigstens ein Parameter bestimmt wird, der für das einem Behandlungsraum zugeführte Wasserstoffperoxid oder das in dem Behandlungsraum befindliche Wasserstoffperoxid charakteristisch ist.

Erfindungsgemäß wird die Sensoreinrichtung derart mit dem Behandlungsraum in Strömungsverbindung gebracht, dass der Parameter für das im Behandlungsraum befindliche Wasserstoffperoxid erfassbar ist. Vorteilhaft ist dabei eine unmittelbare Erfassung des Parameters, bei dem es sich insbesondere um eine Wasserstoffperoxidkonzentration handeln kann, möglich.

Vorteilhaft wird die Strömungsverbindung zwischen dem Behandlungsraum und der Sensoreinrichtung wenigstens zeitweise unterbrochen. Dabei ist vorteilhaft eine Absperreinrichtung vorgesehen, welche die besagte Strömungsverbindung wenigstens zeitweise unterbricht. Vorteilhaft ist diese Absperreinrichtung derart gestaltet, dass sie die Strömungsverbindung im Falle beispielsweise eines Stromausfalls oder dergleichen automatisch schließt.

Auch durch das erfindungsgemäße Verfahren kann trotz der relativ harten Umgebungsbedingungen ein kostengünstiges Messgerät verwendet werden. Daneben können die Konzentrationen in den Sterilisationskammern jederzeit überwacht und kontrolliert werden.

Bei einer vorteilhaften Ausführungsform ist eine Sperreinrichtung zwischen der Sensoreinrichtung und dem Behandlungsraum vorgesehen, welche eine Verunreinigung der Sensoreinrichtung mit Flüssigkeiten (wie beispielsweise Spülmedien oder Säuren) verhindert, jedoch den Zutritt eines gasförmigen Mediums wie insbesondere von Wasserstoffperoxid zu der Sensoreinrichtung ermöglicht. Diese Sperreinrichtung verhindert damit den Zutritt von Flüssigkeiten aus dem Behandlungsraum zu der Sensoreinrichtung. So wäre es möglich, dass die Sensoreinrichtung oberhalb des Behandlungsraums angeordnet ist und über eine Verbindungsleitung mit dem Behandlungsraum verbunden ist. Diese Verbindungsleitung ist in diesem Fall auch die Sperreinrichtung.

Weitere Vorteile und Ausführungsformen ergeben sich aus den beigefügten Zeichnungen:
Darin zeigen:
   - Fig. 1: eine schematische Darstellung einer Vorrichtung zum Behandeln von Behältnissen; und
   - Fig. 2: eine Detaildarstellung einer Vorrichtung zum Behandeln von Behältnissen mit Sensoreinrichtung.

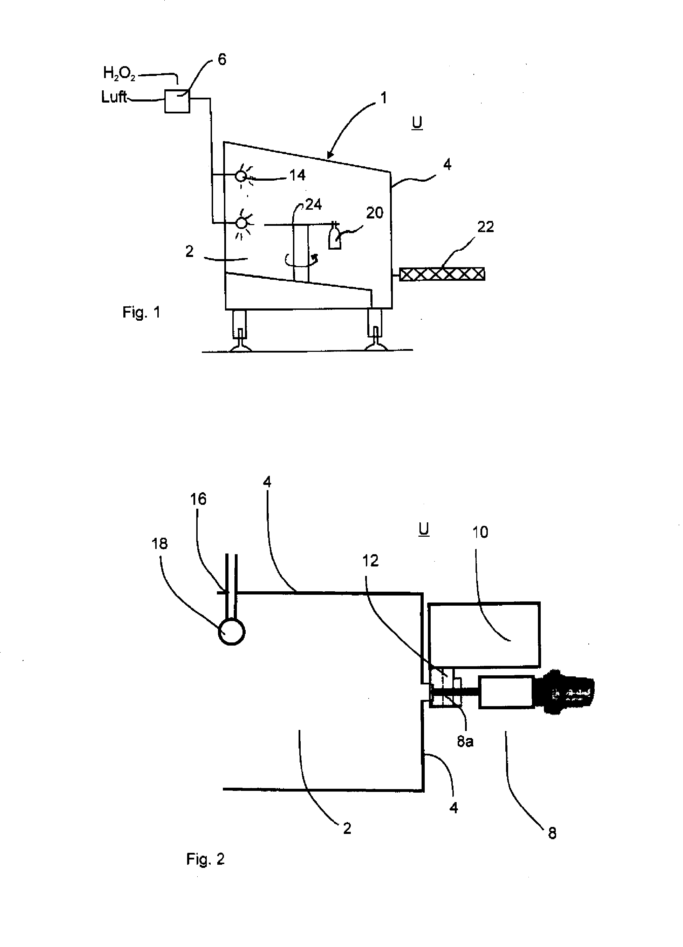

Fig. 1 zeigt eine Vorrichtung 1 zur Behandlung von Behältnissen 20. Dabei ist ein Behandlungsraum 2 vorgesehen, der hier von einer Umfangswandung 4 gegenüber der Umgebung U abgrenzt wird. Im Inneren dieses Behandlungsraums 2 ist eine Transporteinrichtung 24 vorgesehen, welche die Behältnisse 20 transportiert. Daneben könnte jedoch auch eine Behandlungseinheit für Behältnisverschlüsse oder dergleichen vorgesehen sein. Das Bezugszeichen 6 bezieht sich auf eine Erzeugungseinheit zum Erzeugen von Wasserstoffperoxiddampf, der über Zuführeinrichtungen 14 in den Behandlungsraum 2 eingebracht wird. Das Bezugszeichen 22 kennzeichnet einen Auslass für das Sterilisationsmedium. Die der Erfindung zugrunde liegende Aufgabe besteht darin, die H₂O₂ - bzw. allgemein Sterilisationsmittel - Atmosphäre im Inneren des Behandlungsraums zu kontrollieren.

Fig. 2 zeigt eine Detaildarstellung einer erfindungsgemäßen Vorrichtung. Dabei ist hier eine Sensoreinrichtung 10 vorgesehen, die außerhalb der Wand 4 des Behandlungsraums 2 angeordnet ist, und die zum Messen einer Konzentration des H₂O₂ -dient. Diese Sensoreinrichtung 10 steht dabei über eine Verbindungsleitung 12 in Strömungsverbindung mit dem Behandlungsraum 2. Auf diese Weise kann die Sensoreinrichtung die Konzentration des H₂O₂ bestimmen.

Das Bezugszeichen 8 bezieht sich in seiner Gesamtheit auf eine Absperr- bzw. Ventileinrichtung, welche hier einen Ventilkörper 8a aufweist, der die Verbindung 12 zwischen dem Behandlungsraum 2 und der Sensoreinrichtung 10 unterbrechen kann. Auf diese Weise kann sichergestellt werden, dass die Sensoreinrichtung 10 beispielsweise während eines Reinigungsbetriebs nicht mit einem entsprechenden Reinigungsmittel in Kontakt kommt. Das Bezugszeichen 16 bezieht sich auf eine Zuführleitung, um im Behandlungsraum 2 ein Spülmedium zuzuführen und das Bezugszeichen 18 kennzeichnet einen entsprechenden Ausgabekopf wie beispielsweise einen Sprühkopf. Auf diese Weise ist es möglich, eine CIP Reinigung des Behandlungsraums 2 und gegebenenfalls auch der Transporteinrichtung 24 durchzuführen.

In dem Behandlungsraum 2 könnten unterschiedliche Einheiten zum Behandeln von Behältnissen vorgesehen sein wie beispielsweise Sterilisationseinheiten, welche eine Außen- oder Innenwandung des Behältnisses sterilisieren, Fülleinheiten, Spüleinheiten und dergleichen.

Die Anmelderin behält sich vor, weitere in den Anmeldungsunterlagen offenbarte Merkmale als erfindungswesentlich zu beanspruchen, sofern sie einzeln oder in Kombination gegenüber dem Stand der Technik neu sind.

### Bezugszeichenliste

- 1: Vorrichtung
- 2: Behandlungsraum
- 4: Umfangswandung
- 6: Erzeugungseinheit
- 8: Absperr-, Ventileinrichtung
- 8a: Ventilkörper
- 10: Sensoreinrichtung
- 12: Verbindungsleitung
- 14: Zuführeinrichtung
- 16: Zuführleitung
- 18: Ausgabekopf
- 20: Behältnis
- 22: Auslass
- 24: Transporteinrichtung
- U: Umgebung

## Patentansprüche

1. Vorrichtung (1) zur Behandlung von Packmitteln (20) mit einem Behandlungsraum (2), der gegenüber seiner Umgebung wenigstens teilweise abgetrennt ist, mit einer Zuführeinrichtung, welche dem Behandlungsraum Wasserstoffperoxid (H₂O₂) zuführt und mit einer Sensoreinrichtung (10), welche wenigstens einen für das Wasserstoffperoxid (H₂O₂) relevanten Parameter erfasst,
**dadurch gekennzeichnet, dass**
die Sensoreinrichtung (10) derart mit dem Behandlungsraum (2) in Strömungsverbindung bringbar ist, dass der Parameter für das in dem Behandlungsraum (2) befindliche Wasserstoffperoxid von der Sensoreinrichtung erfassbar ist.

2. Vorrichtung (1) nach Anspruch 1,
**dadurch gekennzeichnet, dass**
die Sensoreinrichtung (10) über eine Verbindungsleitung (12) mit dem Behandlungsraum (2) in Verbindung steht.

3. Vorrichtung (1) nach wenigstens einem der vorangegangenen Ansprüche,
**dadurch gekennzeichnet, dass**
eine Absperreinrichtung (8) zum wenigstens teilweise Absperren der Strömungsverbindung zwischen dem Behandlungsraum (2) und der Sensoreinrichtung (10) vorgesehen ist.

4. Vorrichtung (1) nach wenigstens einem der vorangegangenen Ansprüche,
**dadurch gekennzeichnet, dass**
der Parameter eine Konzentration des Wasserstoffperoxids ist.

5. Vorrichtung (1) nach wenigstens einem der vorangegangenen Ansprüche,
**dadurch gekennzeichnet, dass**
die Sensoreinrichtung (10) an einer Außenwandung (4) des Behandlungsraums (2) angeordnet ist.

6. Vorrichtung (1) nach wenigstens einem der vorangegangenen Ansprüche,
**dadurch gekennzeichnet, dass**
der Behandlungsraum (2) zur Aufnahme von Behältnissen (20) dient.

7. Vorrichtung (1) nach wenigstens einem der vorangegangenen Ansprüche,
**dadurch gekennzeichnet, dass**
die Vorrichtung (1) eine Transporteinrichtung (24) aufweist, welche die Behältnisse (20) innerhalb des Behandlungsraums (2) transportiert.

8. Anlage (1) zur Behandlung von Packmitteln mit einer Vorrichtung (1) nach wenigstens einem der vorangegangenen Ansprüche, sowie einer zweiten Behandlungsvorrichtung, wobei die Sensoreinrichtung (10) auch zur Bestimmung wenigstens eines Parameters für das in der zweiten Behandlungsvorrichtung befindliche Wasserstoffperoxid (H₂O₂) dient.

9. Verfahren zur Wasserstoffperoxid (H₂O₂) - Bestimmung, wobei mittels einer Sensoreinrichtung (10) wenigstens ein Parameter bestimmt wird, der für das einem Behandlungsraum (2) zugeführte Wasserstoffperoxid (H₂O₂) charakteristisch ist,
**dadurch gekennzeichnet, dass**
die Sensoreinrichtung (10) derart mit dem Behandlungsraum (2) in Strömungsverbindung gebracht wird, dass der Parameter für das in dem Behandlungsraum (2) befindliche Wasserstoffperoxid erfassbar ist.

10. Verfahren nach Anspruch 9,
**dadurch gekennzeichnet, dass**
die Strömungsverbindung zwischen dem Behandlungsraum (2) und der Sensoreinrichtung (10) wenigstens zeitweise unterbrochen wird.
